# NEW EUROPEAN PATENT SPECIFICATION

(11) **EP 2 806 880 B2**
(45) Date of publication and mention of the opposition decision: **22.04.2020**
(45) Mention of the grant of the patent: 03.05.2017
(21) Application number: 13708527.0
(22) Date of filing: 24.01.2013
(51) Int. Cl.: A61K 31/734, A61K 33/10, A61P 1/04, A61K 33/00, A61K 33/06

(54) **PHARMACEUTICAL COMPOSITION AS A SUBSTANCE FOR ANTIREFLUX ANTACID DRUG**
PHARMAZEUTISCHE ZUSAMMENSETZUNG ALS ANTIREFLUX-MEDIKAMENT
COMPOSITION PHARMACEUTIQUE POUR SUBSTANCE ANTIREFLUX, ANTIACIDE

(30) Priority: 27.01.2012 BY 20120125
(43) Date of publication of application: 03.12.2014
(73) Proprietor: Institute of Bioorganic Chemistry, Minsk 220141 (BY)
(72) Inventor: MAKARENKA VASILEVICH, Mikhail, Minsk 220141 (BY); PETROV TIMOPHEEVICH, Peter, Minsk 220039 (BY); DZIAMID IOSIPHOVICH, Dzmitriy, Brestskiy reg. 225320 (BY); KAZYUCHITS ALEKSANDROVNA, Olga, Minsk 220024 (BY); ILYANOK ALEXEEVNA, Halina, Minsk 220007 (BY); HAURYLAU VLADIMIROVICH, Mikhail, Minsk 220050 (BY); USANAU ALEKSANDROVICH, Siarhei, Minsk 220141 (BY)
(74) Representative: Kuzjukevica, Lucija
(86) International application number: PCT/IB2013/050602
(87) International publication number: WO 2013/111077

(56) References cited:
- WO-A2-03/068246
- WO-A2-03/068246
- BY-C1- 9 674
- BY-C1- 9 675
- GB-A- 1 199 039
- US-A- 5 681 827
- HAMPSON F C ET AL: "Alginate rafts and their characterisation", INTERNATIONAL JOURNAL OF PHARMACEUTICS, ELSEVIER BV, NL, vol. 294, no. 1-2, 27 April 2005 (2005-04-27), pages 137-147, XP027624080, ISSN: 0378-5173 [retrieved on 2005-04-27]

## Description

### Technical Field

The invention relates to the field of medicine, namely, gastroenterology, drugs for the acid-dependent diseases of gastrointestinal tract treatment, especially gastroesophageal reflux disease and peptic ulcer, namely with pharmaceutical composition comprising sodium alginate, calcium carbonate, potassium bicarbonate, magnesium containing compound and hydroxyapatite as the active ingredients.

### Background Art

Wide distribution among drugs of this group have found different polysaccharides containing products forming at interaction with the stomach acid the gel foam or raft, which floats on the surface of the stomach contents thus preventing the release of acidic stomach contents into the esophagus, thus saving the mucosa from further irritation. The analysis of the market of antireflux medicines shows that in different countries represented different drug forms with different combinations raft formative and antacid components. These preparations are significantly vary based on acid neutralizing capacity (ANC), side effects and systemic complications. There are drugs of this type, containing a minimal recommended dose (2 tablets or 1 sachet) 0.52 g of alginic acid or its sodium salt, 0.2 g of sodium bicarbonate and 0.26 g of a mixture of magnesium trisilicate and aluminium hydroxide disclosed in GB 1524740 A (RECKITT & COLMANN PROD LTD) 13.09.1978. To the content of one sachet before use is usually added 20 ml of water and mix. Patent US 4414198 A (MICHAELSON JOSEPH) 08.11.1983 is disclosed rapidly disintegrating in water tablet contains a mixture of alginic acid / pectin, acidic casein and water-soluble carbonate. The amount of alginic acid equals 0, 05-25% (mostly 3-15%) of the weight of the tablet. As disintegrator is stated L-leucine and L-isoleucine at 0.3 - 1.5 parts to 10 parts by weight of the tablet.

Antacid ingredients contain K, Na, Mg, Mn, Ca, Al (hydroxides and oxides, carbonates and bicarbonates). The amount of antacid components is equal 1-75% (1-50% wt.) from the dose, in quantities that provide the ANC value within 5 mEq. ANC of the single dose of drugs used is depended from the composition of ingredients used. For example, 2 tablets (Gaviscon, Canada) WASHINGTON, N. Handbook of Antacids and Anti-reflux Agents. Boca Raton: CRC Press, 1991. p.127. contain 140 mg of sodium bicarbonate and 80 mg of magnesium carbonate and show ANC equal to 4 mEq, while the 2 tablets Gaviscon (USA), containing 140 mg of sodium bicarbonate, 160 mg of aluminium hydroxide and 40 mg of magnesium trisilicate have ANC about 3.7 mEq.

There are known raft-forming compositions based on magnesium alginate and xanthan gum in the presence of potassium bicarbonate, magnesium carbonate, aluminium hydroxide US 5112813 A (RHONE POULENC RORER PHARMA) 12.05.1992 , containing sodium alginate and calcium carbonate, sodium bicarbonate, aluminium hydroxide or magnesium carbonate US 5036057 A (UNIV MELBOURNE) 30.07.1991 , magnesium alginate and potassium bicarbonate, magnesium carbonate, aluminium hydroxide, stabilizer EP 0297109 B (RHONE POULENC RORER INT) 23.08.1995 , and magnesium alginate, sodium carbonate, aluminium hydroxide and magnesium carbonate US 4869902 A (RORER PHARMACEUTICAL CORP) 26.09.1989 , magnesium alginate and magnesium carbonate, aluminium hydroxide, sodium bicarbonate EP 0179858 B (RHONE-POULENC RORER INTERNATIONAL) 01.07.1992. The main negative feature of these compositions is that they use aluminium containing compounds, which limits their application. Admission of aluminium-containing drugs is contraindicated at any renal failure of different origin. At prolonged using of such drugs, Al^{3 +} is accumulated in bone and brain tissues, that increases the risk of developing serious complications of osteomalacia, encephalopathy, renal disease Boutsen Y., et al. Antacid-induced osteomalacia // Clin Rheumatol. 1996. Vol. 15 (1). P. 75-80. Therefore, primarily interest is present antacids not containing aluminium.

The most close to the claimed is a pharmaceutical composition comprising, together with sodium alginate, sodium bicarbonate from 0.16 to 2.60 weight parts relative to the weight of sodium alginate and calcium carbonate from 0.10 to 1.04 parts by weight of the weight of sodium alginate GB 1524740 A (RECKITT) 13.09.1978 . The typical composition of the minimum single dose (2 tablets) of drug: sodium alginate 500 mg, 267 mg of sodium bicarbonate and calcium carbonate 160 mg. The disadvantages of the drug: the composition contains relatively low amount of antacid - ANC of minimal single dose of antacid according to composition ∼ 6.4 mEq and a relatively large amount of sodium - 76.86 mg/g, i.e. in maximal single dose from four tablets, the content of sodium is equal 246 mg. The large amount of sodium is due to content of the sodium-containing ingredients, thus in minimal single dose, along with sodium alginate (500 mg) and sodium bicarbonate (267 mg), is used sodium hydroxide (30 mg) as a pH regulator. Sodium-containing antacids cause fluid retention, which may be non-desirable in some cases of congestive heart failure, arterial hypertension, at renal impairment, and during prolong application or at high doses may cause alkalosis, which is characterized by nausea, lethargy, headache. A side effect due to content of bicarbonate - in a case of overdose may result in stomach bloating.

### Summary of invention

The aim of the present invention was to develop pharmaceutical composition as the substance for antireflux antacids drug.

The aim of the present invention has been achieved by development of pharmaceutical composition as the substance for antireflux antacids, containing sodium alginate and calcium carbonate, and characterized in that it additionally contains potassium bicarbonate, magnesium containing compound, hydroxyapatite in the following ratio mass. %: sodium alginate 26,9-53,0, potassium bicarbonate 7,4-18,5, calcium carbonate 20,4-40,3, magnesium containing compound 3,1-23,5, hydroxyapatite 2,0-14,2. The amounts of potassium bicarbonate and calcium carbonate are sufficient for buoyancy of the gel alginate containing raft without excessive gas generation. Calcium and magnesium facilitate to strengthening of the gel raft formed on the surface of the stomach content due to cross-linking by Ca^{2 +} and Mg^{2 +}cations. Calcium salts cause constipation, therefore inclusion of magnesium containing compound that have laxative effect eliminates the undesirable side effect. As the source of magnesium it is commonly used magnesium hydroxide, magnesium carbonate, magnesium oxide. Magnesium-containing drugs increase mucus-forming and rubber stentnost gastric mucosa that strengthen the protection of the esophageal mucosa from highly aggressive acidic content of stomach in the case of regurgitation.

The pharmaceutical composition of the present invention can be used in the form of chewable tablets, granules, powders or suspensions. The composition may comprises at necessity additional pharmaceutically acceptable ingredients: fillers, suspending agents, binders, preservatives, sweeteners, disintegrating agents, colouring agents, preservatives. To improve the taste and smell of chewable tablets it is possible to use aspartame, sodium saccharine, sorbitol, sodium citrate, and combinations of these, as well as flavors such as "Mint", "Cherry", "Apple", "Raspberry", "Orange", "Vanilla". The tablets are usually prepared using conventional methods and technology of manufacturing tablets. In addition, it is possible to include into composition of the active drug ingredient: analgesics, suppressant, anti-ulcer, anti-fungal, antiseptic, etc.

The claimed invention is explained by example pharmaceutical composition, which does not limit the invention.

### Description of embodiments

The indicated ratios of ingredients are essential for increase of the ANC without over increase of pH value. The testing of composite substances without hydroxyapatite showed an increase of reached pH value above the allowed rate (Table 1, sample 2, 7, 10: The value of 6,3-6,7 and 9,1-9,3, respectively). Hydroxyapatite exhibits the adsorption activity with respect to pepsin and bile acids that also increases the antireflux properties of the drug.

Specific examples of the best compositions of the invention are given in the table 1.

| **Nº** | **Alginate sodium, mg** | **KHCO₃ mg** | **Mg²⁺ mg** | **CaCO₃ mg** | **HA mg** | **Raft g** | **ANA mEq** | **pH** |
|---|---|---|---|---|---|---|---|---|
| 1 | 200 | 55 | 175^{H} | 300 | 15 | 8,4 | 14,4 | 7,6 |
| 2 | 250 | 50 | 45^{O} | 220 | 50 | 8,3 | 10,8 | 6,3 (without HA-9,1) |
| 3 | 250 | 100 | 45^{O} | 110 | 35 | 12,4 | 8,2 | 6,8 |
| 4 | 250 | 50 | 60^{C} | 225 | 50 | 14,1 | 10,3 | 6,5 |
| 5 | 400 | 100 | 26^{O} | 200 | 120 | 13,4 | 8,9 | 6,8 |
| 6 | 400 | 100 | 30^{C} | 200 | 100 | 13,7 | 9,1 | 6,9 |
| 7 | 400 | 100 | 36^{O} | 280 | 100 | 13,5 | 11,6 | 6,7 (without HA -9,3) |
| 8 | 400 | 100 | 35^{O} | 200 | 20 | 17,6 | 9,3 | 7,5 |
| 9 | 400 | 100 | 36^{O} | 200 | 80 | 13,8 | 10,1 | 7,0 |
| 10 | 400 | 100 | 36^{O} | 200 | 40 | 13,2 | 9,7 | 6,4 (without HA -9,2) |
| 11 | 400 | 100 | 100^{C} | 180 | 30 | 17,5 | 10,8 | 7,0 |
| 12 | 400 | 110 | 54^{H} | 172 | 34 | 14,4 | 9,2 | 6,6 |
| 13 | 400 | 110 | 56^{H} | 173 | 35 | 14,9 | 9,3 | 6,7 |
| 14 | 400 | 100 | 80^{O} | 300 | 50 | 16,2 | 15,0 | 7,0 |
| 15 | 420 | 120 | 55^{H} | 200 | 42 | 20,7 | 10,1 | 6,5 |
| 16 | 440 | 121 | 60^{H} | 190 | 36 | 19,1 | 10,3 | 6,8 |
| 17 | 500 | 145 | 67^{H} | 215 | 40 | 19,8 | 11,5 | 6,9 |
| 18 | 600 | 165 | 78^{H} | 255 | 54 | 24,4 | 13,2 | 7,0 |
| where the index: O - magnesium oxide, H-magnesium hydroxide, C- magnesium carbonate | | | | | | | | |

Determination of the sodium content in tablet formulation by atomic emission spectrometry with inductively coupled plasma using spectrometer ARL 3410 + (U.S.) showed sodium levels 14,0-18,6 mg/g, that is 4,1-5,5 times less than in the prototype. The results of *in vitro* testing confirm that composites during interaction with a 0.1 M solution of hydrochloric acid form a alginate-containing gel rafts (volume 19-48 ml) preserving buoyancy during several hours. The weight obtained rafts depends on the quantitative and qualitative content of the ingredients (see table) and equals 8,3-24,4 g. Determination of the acid neutralizing capacities of the claimed substances was conducted under standard conditions of *in vitro* model using modified method RIGO.

The samples of pharmaceutical composition used as substance for antireflux antacids preparation demonstrate more higher values as compared to the prototype ANC 8,2-15,0 mEq, that corresponds to requirements of the modern pharmacopoeia (normal: ANC ≥ 5 mEq).

Experimental data for testing of pharmacological composites confirm evident raft formation and high ANC of admissible at achievable pH of solutions (normal: pH ≤ 7,0).

### Example 1.

A pharmaceutical composition as the substance for antireflux antacids.

To obtain the substance it is necessary to mix 120 g of potassium bicarbonate, 200 g of calcium carbonate, 56 g of magnesium hydroxide, 43 g of hydroxyapatite and 420 g of sodium alginate. The resulting mixture is thoroughly mixed to obtain a homogeneous powder. As additional components it is added 200 g of macrogol, 60 g of copovidone, 1.5 g of saccharin, 2.4 g of flavor "Cherry", 367.1 g of sorbitol, 15 g of magnesium stearate. After mixing and tableting of the mixture of the components, the tablets were obtained with biconvex shape and diameter of 12 mm, tablet weight 0,7502 ± 0,0034 g. Sodium 16.27 mg/g. Tested *in vitro* for a single dose (2 chewable tablets) showed that the weight of the resulting alginate containing raft was equal to 23.3 g, and the ANC was 10.2 mEq.

## Claims

1. Pharmaceutical composition comprising as the active ingredients sodium alginate and calcium carbonate, **characterized in that** it additionally comprises potassium bicarbonate, magnesium containing compound and hydroxyapatite, wherein the mass ratio of sodium alginate is 26,9-53,0%, potassium bicarbonate 7,4-18,5%, calcium carbonate 20,4-40,3%, magnesium containing compound 3,1-23,5% and hydroxyapatite 2,0-14,2%, and wherein magnesium containing compound is selected from the list: magnesium hydroxide, magnesium carbonate or magnesium oxide.

## Patentansprüche

1. Pharmazeutische Zusammensetzung, die als Wirkstoffe Natriumalginat und Calciumcarbonat umfaßt, **dadurch gekennzeichnet, daß** sie zusätzlich Kaliumbicarbonat, Magnesium enthaltenden Verbindung und Hydroxyapatit umfaßt, wobei das Massenverhältnis von Natriumalginat ist 26,9-53,0%, Kaliumbicarbonat 7,4-18,5%, Calciumcarbonat 20,4-40,3%, Magnesium enthaltenden Verbindung 3,1 -23,5% und Hydroxyapatit 2,0-14,2%, und wobei Magnesium enthaltenden Verbindung aus der Liste ausgewählt ist: Magnesiumhydroxid, Magnesiumcarbonat oder Magnesiumoxid.

## Revendications

1. Une composition pharmaceutique comprenant en tant qu'ingrédients actifs de l'alginate de sodium et de carbonate de calcium, **caractérisé en ce qu'**il comprend en outre du bicarbonate de potassium, du magnésium contenant un composé et de l'hydroxyapatite, le rapport en masse d'alginate de sodium est 26,9-53,0 %, bicarbonate de potassium 7,4-18,5 %, 20,4-40,3 % de carbonate de calcium, du magnésium contenant un composé 3,1-23,5 % et hydroxyapatite 2,0-14,2 %, et ledit composé contenant du magnésium est sélectionnée dans la liste: hydroxyde de magnésium, le carbonate de magnésium ou de l'oxyde de magnésium.
